# EUROPÄISCHE PATENTSCHRIFT

(11) **EP 0 383 900 B1**
(45) Veröffentlichungstag und Bekanntmachung des Hinweises auf die Patenterteilung: **26.05.1993**
(21) Anmeldenummer: 89910083.8
(22) Anmeldetag: 30.08.1989
(51) Int. Cl.: A61M 25/02

(54) **HALTERUNG FÜR MEDIZINISCHE INSTRUMENTE, INSBESONDERE KATHETER, SOWIE VERFAHREN ZU DEREN HERSTELLUNG**
HOLDER FOR MEDICAL INSTRUMENTS, IN PARTICULAR CATHETERS, AND METHOD FOR MAKING THE SAME
FIXATION POUR INSTRUMENTS MEDICAUX, NOTAMMENT CATHETERS, ET METHODE DE FABRICATION DE LAQUELLE

(30) Priorität: 02.09.1988 DE 3829896
(43) Veröffentlichungstag der Anmeldung: 29.08.1990
(73) Patentinhaber: ACONVEST AG, CH-4001 Basel (CH)
(72) Erfinder: PIWONKA, Peter, D-8000 München 71 (DE)
(74) Vertreter: Vogeser, Werner, Dipl.-Ing.
(86) Internationale Anmeldenummer: EP8901018
(87) Internationale Veröffentlichungsnummer: WO9002578

(56) Entgegenhaltungen:
- EP-A- 0 247 571
- DE-U- 8 690 058
- US-A- 4 726 716

## Beschreibung

Die Erfindung betrifft eine Halterung für medizinische Instrumente, insbesondere Katheter, sowie ein Verfahren zu deren Herstellung.

Aus der DE-U-86 90 058.7 sind Halterungen verschiedener Ausführungsform bekannt, die z. B. aus einem Basisstreifen und einer darauf angeordneten klappbaren Lasche bestehen, die am einen Ende mit dem Basisstreifen fest verbunden ist. Auf der Oberseite der Lasche befinden sich zwei voneinander beabstandete Klettverschlußabschnitte. Zwischen diesen ist ein elastisches Klebstoffpolster aufgebracht, auf das das zu haltende Instrument aufgelegt und durch Verbinden der Klettverschlußabschnitte der Lasche gehalten werden kann. Eine solche Halterung hat sich zwar in der Praxis bewährt, jedoch ist die Herstellung kostspielig, da insbesondere die Fertigung der Lasche mehrere Arbeitsgänge und teures Material erfordert.

Aus der US-A-3,834,380 ist eine ähnliche Halterung bekannt, bei der zwischen den Klettverschlußabschnitten ein längsgeschlitztes Klemmrohr zur Aufnahme eines zu haltenden Instruments befestigt ist.

Aus der US-A-4,726,716 ist eine weitere Halterung bekannt, bei der ein Klettverschlußabschnitt auf dem Basisstreifen und ein weiterer auf der Lasche befestigt ist, die an dem mit dem Basisstreifen verbundenen Ende einen Ausschnitt für einen Katheder mit einem Abzweigkanal aufweist.

Aus der DE-U-87 10 021.5 schließlich ist eine Halterung bekannt, bei der die Oberseite des Basisstreifens und die Unterseite der Lasche mit einer Klebstoffschicht versehen ist. Die Lasche steht normalerweise nach oben und ist mit einer doppelt gefalteten Folie versehen, um ein unbeabsichtigtes Haften der Lasche am Basisstreifen zu verhindern.

All diese weiteren bekannten Folien sind in der Herstellung vor allem deshalb zu teuer, weil eine Serienherstellung kaum möglich ist.

Der Erfindung liegt die Aufgabe zugrunde, eine einfach und sicher anwendbare Halterung zu schaffen, die möglichst einfach hergestellt werden kann und ein Verfahren zu deren Herstellung anzugeben.

Gelöst wird diese Aufgabe durch die im Anspruch 1 angegebenen Merkmale. Zweckmäßige Ausgestaltungen der Erfindung ergeben sich aus den Unteransprüchen. Ein erfindungsgemäßes Verfahren zur Herstellung ist im Anspruch 5 angegeben.

Der wesentliche Grundgedanke, der der Erfindung zugrunde liegt, besteht darin, zwei einseitig mit Klebstoff beschichtete und mit einer Abdeckfolie versehene Klebebandstreifen am einen Ende einander überlappend miteinander zu verkleben, wobei beim unteren Klebebandstreifen die Klebstoffschicht unten und beim oberen Klebebandstreifen oben zu liegen kommt. Dabei wird im überlappten Bereich das eine Ende des unteren Klebebbandstreifens klebstofffrei belassen. Bei der Verwendung der Halterung wird zunächst die Abdeckfolie des unteren Klebebandstreifens abgezogen und die Halterung am Patienten befestigt. Danach wird die Abdeckfolie des oberen Klebebandstreifens abgezogen, das Instrument etwa auf das eine Ende des oberen Klebebandstreifen gelegt, und das andere Ende des oberen Klebebandstreifens, der als klappbare Lasche dient, über das Instrument geklappt. Das Ende des unteren Klebebandstreifens, das im überlappten Bereich der beiden Klebebandstreifen Klebstoff freibelassen wurde, gewährleistet, daß Abschälkräfte vom Instrument nicht unmittelbar auf die Kante des einen Endes des unteren Klebebandstreifens wirken. Die seitlichen Einkerbungen am oberen, als Lasche dienenden Klebebandstreifen, die im geklappten Zustand der Lasche über dem Instrument zu liegen kommen, erhöhen die Haftwirkung, da das Instrument im Bereich der Einkerbungen seitliche Bewegungen ausführungen kann, die nicht unmittelbar auf die Halterung wirken. Dieser Effekt läßt sich noch dadurch verbessern, daß die Klebstoffschicht der Lasche zumindest in diesem Bereich in Form von Querstreifen ausgebildet ist.

Um die Haftung noch weiter zu verbessern, können die Ecken aller freiliegenden Enden der Klebebandstreifen abgeschrägt sein.

Die Herstellung von Halterungen dieser Ausgestaltung ist besonders einfach, da zwei mit Klebstoff beschichtete und mit einer Abdeckfolie versehene endlose Klebebandstreifen verwendet werden können, die einander in Längsrichtung teilweise überlappend mit den klebstoffschichtfreien Seiten einander zugewandt übereinandergelegt werden. Beide Klebebandstreifen werden im überlappten Bereich beabstandet zu einer Längskante des unten liegenden Klebebandstreifens miteinander verklebt. Aus den beiden verbundenen Klebebandstreifen können dann Halterungen in der endgültigen Form ausgestanzt werden.

Die Erfindung wird nachstehend anhand der Figuren 1 bis 3 beispielsweise erläutert:

Es zeigen:
Figuren 1 und 2 Seitenansichten der Halterung mit ungeklappter und geklappter Lasche und
Figur 3 eine Aufsicht der Halterung der Figur 2.

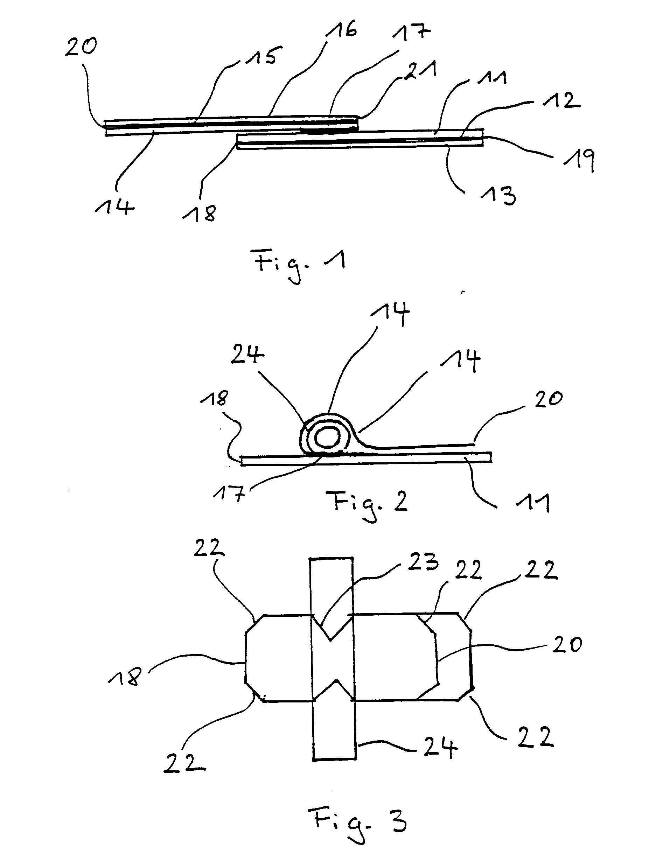

Die Darstellungen insbesondere der Figuren 1 und 2 sind nur schematisch, und die Dicken der verschiedenen Schichten sind zur besseren Darstellung überdimensioniert gezeigt.

Die Halterung besteht, wie Figur 1 zeigt, aus einem unteren Klebebandstreifen 11, der auf der Unterseite eine Klebstoffschicht 12 aufweist, die mit einer Abdeckfolie 13 versehen ist, und einem oberen Klebebandstreifen 14, der als klappbare Lasche dient und der auf der Oberseite eine Klebstoffschicht 15 hat, die mit einer Abdeckfolie 16 versehen ist. Die Lasche 14 ist mit dem Klebebandstreifen 11 im Bereich des einen Endes 21 mit einer Klebstoffschicht 17 derart versehen, daß die Oberseite des Klebebandstreifens 11 im Bereich des einen Endes 18 nicht mit der Lasche 14 verbunden ist. Die Lasche 14 ist derart befestigt, daß sie abgewandt zum anderen Ende 19 des Klebebandstreifens 11 gerichtet ist.

Aus den Fig. 2 und 3 ist ersischtlich, wie z. B. ein schlauchförmiges Instrument 24 gehalten werden kann. Hierzu wird zunächst die Abdeckfolie 16 der Lasche 14 abgezogen, und das Instrument 24 wird auf den Klebebandstreifen 11 aufgelegt, wobei es teilweise im Bereich des anderen Endes 21 der Lasche 14 auf dieser aufliegt. Danach wird die Lasche 14 über des Instrument geklappt und mit der Oberseite des Klebebandstreifens 11 verbunden, so daß der Klebebandstreifen 11 im Bereich des Endes 18 über die Lasche 14 vorsteht.

Wie Figur 3 zeigt, hat die Lasche 14 seitliche Einkerbungen 23, die im geklappten Zustand der Lasche über dem Instrument 24 und im ungeklappten Zustand so liegen, daß deren eine Seite mit Abschrägungen 22 am einen Ende 18 des Klebebandstreifens 11 übereinstimmen, was den Vorteil hat, daß die Einkerbungen 23 und die Abschrägungen 22 am einen Ende 18 zusammen gestanzt werden können.

## Patentansprüche

1. Halterung für medizinische Instrumente, bestehend aus zwei einander am einen Ende (18, 21) überlappenden Klebebandstreifen (11, 14), von denen der untere (11) auf der Unterseite und der obere (14) auf der Oberseite eine mit einer abziehbaren Abdeckfolie (13, 16) versehene Klebeschicht (12, 15) aufweist, der obere Klebebandstreifen (14), der als eine über ein Instrument (24) klappbare Lasche dient, mit dem einen Ende (21) im Überlappungsbereich gegenüber dem einen Ende (18) des unteren Klebebandstreifens (11) versetzt fest verbunden ist, wobei das eine Ende (21) des oberen Klebebandstreifens (14) nach Abziehen der Abkeckfolie (16) als Auflagefläche für das zu haltende Instrument (24) dient, und der obere Klebebandstreifen in dem Bereich seitliche Einkerbungen (23) aufweist, in dem er über dem Instrument (24) zu liegen kommt.

2. Halterung für medizinische Instrumente nach Anspruch 1,
**dadurch gekennzeichnet,** daß
die Ecken (22) der Klebebandstreifen (11, 14) abgeschrägt sind.

3. Halterung für medizinische Instrumente nach Anspruch 2,
**dadurch gekennzeichnet,** daß
die eine Seite der Einkerbungen (23) im nichtgeklappten Zustand des als Lasche dienenden oberen Klebebandstreifens (14) mit den Abschrägungen (22) am einen Ende (18) des unteren Klebebandstreifens (11) übereinstimmt.

4. Halterung für medizinische Instrumente nach einem der Ansprüche 1 - 3,
**dadurch gekennzeichnet,** daß
die Klebstoffschicht (15) des oberen Klebebandstreifens (14) teilweise in Form von Querstreifen ausgebildet ist.

5. Verfahren zur Herstellung von Halterungen nach einem der Ansprüche 1 - 4,
**dadurch gekennzeichnet**, daß
zwei endlose, einseitig mit Klebstoff und einer abziehbaren Abdeckfolie beschichtete Klebebänder einander in Längsrichtung teilweise überlappend mit den klebstoffschichtfreien Seiten einander zugewandt übereinandergelegt werden, daß die Klebebänder im überlappten Bereich beabstandet zur einen Längskante des unten liegenden Klebebands miteinander verklebt werden und daß die Halterungen in ihrer endgültigen Form aus den beiden Klebebändern ausgestanzt werden.

## Claims

1. Holder for medical instruments, consisting of two adhesive tape strips (11, 14) which overlap one another at one end (18, 21), of which the lower (11) has on the underside and the upper (14) on the upper side an adhesive layer (12, 15) provided with a cover film (13, 16) which can be peeled off; the upper adhesive tape strip (14), which serves as a tab which can be folded over an instrument (24), is firmly bonded to one end (21) in the overlapping region so as to be offset with respect to one end (18) of the lower adhesive tape strip (11), one end (21) of the upper adhesive tape strip (14), after peeling off the cover film (16), serving as support surface for the instrument (24) to be held, and the upper adhesive tape strip having lateral notches (23) in the region in which it comes to lie over the instrument (24).

2. Holder for medical instruments according to Claim 1, characterised in that the corners (22) of the adhesive tape strips (11, 14) are sloped.

3. Holder for medical instruments according to Claim 2, characterised in that, in the unfolded state of the upper adhesive tape strip (14) serving as a tab, one side of the notches (23) coincides with the slopes (22) at one end (18) of the lower adhesive tape strip (11).

4. Holder for medical instruments according to one of Claims 1 - 3, characterised in that the adhesive layer (15) of the upper adhesive tape strip (14) is designed partly in the form of transverse strips.

5. Method for making holders according to one of Claims 1 - 4, characterised in that two continuous adhesive tapes coated with adhesive on one side and with a cover film which can be peeled off are laid one on the other partly overlapping one another in the longitudinal direction and with the adhesive layer-free sides directed towards one another, in that the adhesive tapes are adhesively bonded to one another in the overlapped region at a distance from a longitudinal edge of the lower adhesive tape and in that the holders in their final shape are punched out of the two adhesive tapes.

## Revendications

1. Fixation pour instruments médicaux, constituée de deux bandes de ruban adhésif (11, 14) se chevauchant l'une l'autre à une extrémité (18, 21), dont la bande inférieure (11) présente sur le côté inférieur, et la bande supérieure (14) sur le côté supérieur, une couche adhésive (12, 15) pourvue d'un film de recouvrement pelable (13, 16), la bande de ruban adhésif supérieure (14) qui sert de languette pouvant être rabattue sur un instrument (24), est reliée de manière fixe à la bande de ruban adhésif inférieure, par une extrémité (21) décalée par rapport à une extrémité (18) de la bande de ruban adhésif inférieure (11) dans la zone de chevauchement, cette extrémité (21) de la bande de ruban adhésif supérieure (14), après retrait du film de recouvrement (16), servant comme surface d'appui pour l'instrument (24) à fixer, et la bande de ruban adhésif supérieure présentant des encoches latérales (23), dans la zone dans laquelle elle vient reposer sur l'instrument (24).

2. Fixation pour instruments médicaux selon la revendication 1, caractérisée en ce que les coins (22) des bandes de ruban adhésif (11, 14) sont biseautés.

3. Fixation pour instruments médicaux selon la revendication 2, caractérisée en ce que l'un des côtés des encoches (23), dans l'état non rabattu de la bande de ruban adhésif supérieure (14) servant comme languette, coïncide avec les parties biseautées (22) à une extrémité (18) de la bande de ruban adhésif inférieure (11).

4. Fixation pour instruments médicaux selon l'une des revendications 1 à 3, caractérisée en ce que la couche d'adhésif (15) de la bande de ruban adhésif supérieure (14) est réalisée en partie sous forme de bandes transversales.

5. Procédé de fabrication de fixations selon l'une des revendications 1 à 4, caractérisé en ce que deux rubans adhésifs sans fin revêtus d'un côté avec de l'adhésif et un film de recouvrement pelable sont superposés l'un à l'autre en se chevauchant partiellement dans la direction longitudinale, avec les côtés ne comportant pas de couche d'adhésif tournés l'un vers l'autre, en ce que les rubans adhésifs sont collés l'un avec l'autre dans la zone de chevauchement, à distance d'un bord longitudinal du ruban adhésif situé dessous, et en ce que les fixations sont découpées à la matrice dans leur forme définitive à partir des deux rubans adhésifs.
